# EUROPEAN PATENT APPLICATION

(11) **EP 4 489 013 A1**
(43) Date of publication of application: **08.01.2025**
(21) Application number: 23763366.4
(22) Date of filing: 24.02.2023
(51) Int. Cl.: G16C 20/70

(54) **INFORMATION PROCESSING DEVICE, INFORMATION PROCESSING SYSTEM, PROGRAM, AND MATERIAL COMPOSITION SEARCHING METHOD**

(30) Priority: 01.03.2022 JP 2022031084
(71) Applicant: Resonac Corporation, Tokyo 105-7325 (JP)
(72) Inventor: SAKAGUCHI, Suguru, Tokyo 105-8518 (JP); KAKUDA, Kohsuke, Tokyo 105-8518 (JP); OKUNO, Yoshishige, Tokyo 105-8518 (JP)
(74) Representative: Strehl Schübel-Hopf & Partner
(86) International application number: PCT/JP2023/006789
(87) International publication number: WO 2023/167107

(57) **Abstract**

An information processing device for supporting creation of an Ising model for causing an annealing type computer to solve a combinatorial optimization problem, includes a calculation device that calculates a relationship between an explanatory variable of a function to be converted into the Ising model and a physical property of a mixed material, by a trained machine learning model for predicting the physical property of the mixed material and described using the explanatory variable, a determination device that determines an optimal value and a tolerable variation width of the explanatory variable for the target physical property, based on the relationship between the explanatory variable and the physical property of the mixed material, and an output device that outputs the determined optimal value of the explanatory variable as a target value, and outputs a weighting coefficient of the explanatory variable based on the determined tolerable variation width of the explanatory variable.

## Description

### TECHNICAL FIELD

The present disclosure relates to information processing devices, information processing systems, programs, and material composition searching methods.

### BACKGROUND ART

For example, there is a combinatorial optimization problem for selecting an optimal combination from combinations of various elements, such as searching for a material composition having an optimal physical property or the like, for example. Because the number of combinations increases explosively as the number of elements increases, it may not be possible to solve the combinatorial optimization problem within a realistic time. For example, in a case where 100 kinds of materials are combined in increments of 1% to generate mixed materials, the number of combinations become 5 × 10⁵⁸.

An annealing machine using an Ising model has been proposed as an architecture specialized for solving such a combinatorial optimization problem. The annealing machine can efficiently solve the combinatorial optimization problem converted into the Ising model.

Conventionally, there is a known technique for optimizing the thermophysical properties of mixed refrigerants, using a computer architecture specialized for the combinatorial optimization problem (refer to Non-Patent Document 1, for example).

### PRIOR ART DOCUMENTS

### NON-PATENT DOCUMENTS

Non-Patent Document 1: Takeshi Shioga et al., "Optimization of Thermosphysical Properties for Mixed Refrigerants with Digital Annealer", Proceedings of the Thermal Engineering Conference 2019, Japan Society of Mechanical Engineers No.19-303, [2019.10.12-13, Nagoya]

### DISCLOSURE OF THE INVENTION

### PROBLEM TO BE SOLVED BY THE PRESENT INVENTION

For example, a user desiring to solve a combinatorial optimization problem of a material composition asymptotically approaching (approximating) a target physical property using an annealing machine needs to prepare a function to be converted into an Ising model. The function to be converted into the Ising model is described so as to include an explanatory variable group, and it is necessary to satisfy a constraint condition that the variable group is an optimization result (an optimal composition of the mixed material) when the function has a minimum value. In addition, the function to be converted into the Ising model includes arbitrary constants, such as target values of explanatory variables, weighting coefficients, or the like, and these constants need to be adjusted.

As described above, in the case where the combinatorial optimization problem of the material composition asymptotically approaching (approximating) the target physical property is solved using the annealing machine, there is a problem in that it takes time and effort to create the function to be converted into the Ising model.

One object of the present disclosure is to provide an information processing device, an information processing system, a program, and a material composition searching method that can reduce the time and effort required to create an Ising model for causing an annealing type computer to solve a combinatorial optimization problem of a material composition asymptotically approaching a target physical property.

### MEANS FOR SOLVING THE PROBLEM

The present disclosure includes the following configurations.
[1] An information processing device configured to support creation of an Ising model for causing an annealing type computer to solve a combinatorial optimization problem of a material composition asymptotically approaching a target physical property, characterized in that there are provided:
   a calculation device configured to calculate a relationship between one or more explanatory variables of a function to be converted into the Ising model and a physical property of a mixed material, by a trained machine learning model that is for predicting the physical property of the mixed material and is described using the one or more explanatory variables;
   a determination device configured to determine an optimal value and a tolerable variation width of the one or more explanatory variables for the target physical property, based on the relationship between the one or more explanatory variables and the physical property of the mixed material; and
   an output device configured to output the determined optimal value of the one or more explanatory variables as a target value of the one or more explanatory variables of the function, and output a weighting coefficient of the one or more explanatory variables of the function based on the determined tolerable variation width of the one or more explanatory variables.
[2] The information processing device of [1], characterized in that the one or more explanatory variables are characteristics of the mixed material describable by a weighted average of a ratio of the material composition.
[3] The information processing device of [1] or [2], characterized in that the output device outputs a smaller weighting coefficient for the explanatory variable having a larger tolerable variation width, and outputs a larger weighting coefficient for the explanatory variable having a smaller tolerable variation width.
[4] The information processing device of any one of [1] to [3], characterized in that the determination device determines the tolerable variation width for each of the one or more explanatory variables, based on a threshold value of a tolerable error of the target physical property.
[5] The information processing device of any one of [1] to [4], characterized in that the machine learning model is trained of a relationship between a characteristic of the mixed material describable by a weighted average of a ratio of the material composition and the physical property of the mixed material, using experimental data.
[6] The information processing device of any one of [1] to [5], characterized in that there is further provided:
   a conversion device configured to convert the function, to which the target value of the one or more explanatory variables and the weighting coefficient of the one or more explanatory variables output by the output device are substituted, into the Ising model.
[7] An information processing system including an annealing type computer using an Ising model, and an information processing device configured to support creation of the Ising model for causing the computer to solve a combinatorial optimization problem of a material composition asymptotically approaching a target physical property, characterized in that there are provided:
   a calculation device configured to calculate a relationship between one or more explanatory variables of a function to be converted into the Ising model and a physical property of a mixed material, by a trained machine learning model that is for predicting the physical property of the mixed material and is described using the one or more explanatory variables;
   a determination device configured to determine an optimal value and a tolerable variation width of the one or more explanatory variables for the target physical property, based on the relationship between the one or more explanatory variables and the physical property of the mixed material;
   an output device configured to output the determined optimal value of the one or more explanatory variables as a target value of the one or more explanatory variables of the function, and output a weighting coefficient of the one or more explanatory variables of the function based on the determined tolerable variation width of the one or more explanatory variables;
   a conversion device configured to convert the function, to which the target value of the one or more explanatory variables and the weighting coefficient of the one or more explanatory variables output by the output device are substituted, into the Ising model;
   an optimal solution calculation device configured to calculate an optimal solution of the material composition asymptotically approaching the target value, using the Ising model; and
   a display device configured to display the optimal solution of the material composition asymptotically approaching the target value.
[8] A program which causes an information processing device configured to support creation of an Ising model for causing an annealing type computer to solve a combinatorial optimization problem of a material composition asymptotically approaching a target physical property, to function as:
   a calculation device configured to calculate a relationship between one or more explanatory variables of a function to be converted into the Ising model and a physical property of a mixed material, by a trained machine learning model that is for predicting the physical property of the mixed material and is described using the one or more explanatory variables;
   a determination device configured to determine an optimal value and a tolerable variation width of the one or more explanatory variables for the target physical property, based on the relationship between the one or more explanatory variables and the physical property of the mixed material; and
   an output device configured to output the determined optimal value of the one or more explanatory variables as a target value of the one or more explanatory variables of the function, and output a weighting coefficient of the one or more explanatory variables of the function based on the determined tolerable variation width of the one or more explanatory variables.
[9] A material composition searching method for an information processing system including an annealing type computer using an Ising model, and an information processing device configured to support creation of the Ising model for causing the computer to solve a combinatorial optimization problem of a material composition asymptotically approaching a target physical property, characterized in that there are provided:
   calculating a relationship between one or more explanatory variables of a function to be converted into the Ising model and a physical property of a mixed material, by a trained machine learning model that is for predicting the physical property of the mixed material and is described using the one or more explanatory variables;
   determining an optimal value and a tolerable variation width of the one or more explanatory variables for the target physical property, based on the relationship between the one or more explanatory variables and the physical property of the mixed material;
   outputting the determined optimal value of the one or more explanatory variables as a target value of the one or more explanatory variables of the function, and output a weighting coefficient of the one or more explanatory variables of the function based on the determined tolerable variation width of the one or more explanatory variables;
   converting the function, to which the target value of the one or more explanatory variables and the weighting coefficient of the one or more explanatory variables output by the output device are substituted, into the Ising model;
   calculating an optimal solution of the material composition asymptotically approaching the target value, using the Ising model; and
   displaying the optimal solution of the material composition asymptotically approaching the target value.

### EFFECTS OF THE INVENTION

According to the present disclosure, it is possible to provide an information processing device, an information processing system, a program, and a material composition searching method that can reduce the time and effort required to create an Ising model for causing an annealing type computer to solve a combinatorial optimization problem of a material composition asymptotically approaching a target physical property.

### BRIEF DESCRIPTION OF THE DRAWINGS

[FIG. 1] FIG. 1 is a configuration diagram of an example of an information processing system according to the present embodiment.
[FIG. 2] FIG. 2 is a hardware configuration diagram of an example of a computer according to the present embodiment.
[FIG. 3] FIG. 3 is a configuration diagram of an example of an information processing system according to the present embodiment.
[FIG. 4] FIG. 4 is a flow chart illustrating an example of a processing procedure of a material composition searching method using the information processing system according to the present embodiment.
[FIG. 5] FIG. 5 is an explanatory diagram of an example of a function to be converted into an Ising model.
[FIG. 6A] FIG. 6A is an explanatory diagram of an example of a problem to be solved as a combinatorial optimizing problem.
[FIG. 6B] FIG. 6B is an explanatory diagram of the example of the problem to be solved as the combinatorial optimizing problem.
[FIG. 6C] FIG. 6C is an explanatory diagram of the example of the problem to be solved as the combinatorial optimizing problem.
[FIG. 7] FIG. 7 is a diagram for explaining coordination between combinatorial optimization and machine learning.
[FIG. 8] FIG. 8 is a structural diagram illustrating an example of material information.
[FIG. 9] FIG. 9 is a structural diagram illustrating an example of experimental data.
[FIG. 10] FIG. 10 is a diagram of an example of a relationship between a calculated explanatory variable Xᵢ and a variable Z.
[FIG. 11] FIG. 11 is a diagram of an example for explaining an optimal value and a tolerable variation width of the explanatory variable Xᵢ.
[FIG. 12] FIG. 12 is an explanatory diagram of an example of a process for determining a weighting coefficient Aᵢ of the explanatory variable Xᵢ.
[FIG. 13] FIG. 13 is a diagram of an example for explaining a relationship between a threshold value C of a tolerable error from a minimum variable Z and a shape of an explanatory variable region of a mixed material asymptotically approaching a target physical property.

### MODE OF CARRYING OUT THE INVENTION

Next, embodiments of the present invention will be described in detail. The present invention is not limited to the following embodiments.

### <System Configuration>

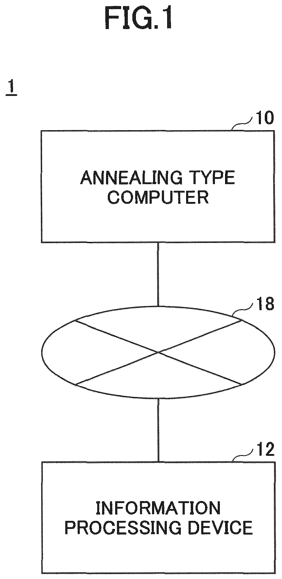

FIG. 1 is a configuration diagram of an example of an information processing system according to the present embodiment. An information processing system 1 illustrated in FIG. 1 includes an annealing type computer 10, and an information processing device 12. The annealing type computer 10 and the information processing device 12 are communicably connected via a communication network 18, such as a local area network (LAN), the Internet, or the like, and a data communication can be performed therebetween.

The annealing type computer 10 is an example of a device that solves a combinatorial optimization problem using an Ising model. The annealing type computer 10 is an annealing machine using the Ising model, for example. The annealing type computer 10 may be implemented by a quantum computer, or may be implemented by a digital annealer (registered trademark) or the like which is a computer architecture achieving an annealing method using a digital circuit.

The annealing machine solves the combinatorial optimization problem that is reduced to the Ising model, by a convergence behavior of the Ising model. The Ising model is a statistical mechanics model representing the behavior of a magnetic body. The Ising model has properties such that a state of spin is updated so that an energy (Hamiltonian) becomes a minimum due to an interaction between the spins of the magnetic material, and the energy finally becomes the minimum. The annealing machine reduces the combinatorial optimization problem to the Ising model, obtains the state in which the energy becomes the minimum, and can obtain the state as an optimal solution of the combinatorial optimization problem.

The information processing device 12 is a device operated by a user, such as a PC, a tablet terminal, a smartphone, or the like. The information processing device 12 receives an input of information required for causing the annealing type computer 10 to solve the combinatorial optimization problem that is reduced to the Ising model, from a user, and causes the annealing type computer 10 to solve the Ising model.

The information required for causing the annealing type computer 10 to solve the combinatorial optimization problem that is reduced to the Ising model, includes information on a function to be converted into the Ising model. The information processing device 12 supports creation of a function to be converted into the Ising model, with respect to the user, and supports creation of the Ising model, as will be described later.

In addition, the information processing device 12 receives the optimal solution of the combinatorial optimization problem solved by the annealing type computer 10, and outputs the optimal solution in a manner checkable by the user, such as displaying the optimal solution on a display device or the like.

The information processing system 1 of FIG. 1 is merely an example, and the user may access and use the information processing device 12 from a user terminal (not illustrated) connected to the information processing device 12 via the communication network 18.

Moreover, the annealing type computer 10 may be implemented as a cloud computing service. For example, the annealing type computer 10 may be available by calling an application programming interface (API) via the communication network 18.

Furthermore, the annealing type computer 10 is not limited that implemented as the cloud computing service, and may be implemented as an on-premise service or may be a service operated by another company. The annealing type computer 10 may be implemented by a plurality of computers.

In a mode in which the user accesses and utilizes the information processing device 12, the information processing device 12 may be implemented as a cloud computing service, or may be implemented as an on-premise service, or may be a service operated by another company, or may be implemented by a plurality of computers. Of course, various system configuration examples of the information processing system 1 of FIG. 1 are possible depending on the application and purpose.

### <Hardware Configuration>

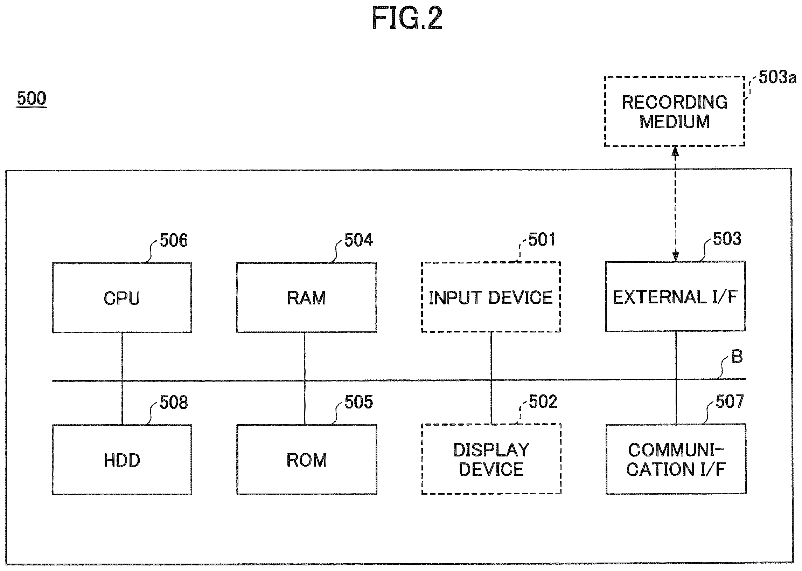

The information processing device 12 of FIG. 1 is implemented by a computer 500 having a hardware configuration illustrated in FIG. 2, for example.

FIG. 2 is a hardware configuration diagram of an example of a computer according to the present embodiment. The computer 500 of FIG. 2 includes an input device 501, a display device 502, an external I/F 503, a RAM 504, a ROM 505, a CPU 506, a communication I/F 507, a HDD 508, or the like, which are connected to each other via a bus B. The input device 501 and the display device 502 may be utilized in a state where the two are connected to each other.

The input device 501 is a touchscreen panel, operation keys and buttons, a keyboard, a mouse, or the like used by the user to input various signals. The display device 502 is configured by a display that displays a screen, such as a liquid crystal display, an organic EL display, or the like, or a speaker or the like that outputs sound data, such as voice, sound, or the like. The communication I/F 507 is an interface used by the computer 500 to perform the data communication.

In addition, the HDD 508 is an example of a nonvolatile storage device that stores programs and data. The stored programs and data include an OS which is basic software for controlling the entire computer 500, applications for providing various functions on the OS, or the like. The computer 500 may utilize a drive device (for example, a solid state drive: SSD or the like) using a flash memory as a storage medium in place of the HDD 508.

The external I/F 503 is an interface with respect to an external device. The external device includes a recording medium 503a or the like. Hence, the computer 500 can perform a read from and/or a write to the recording medium 503a, via the external I/F 503. The recording medium 503a includes a flexible disk, a CD, a DVD, a SD memory card, a USB memory, or the like.

The ROM 505 is an example of a nonvolatile memory (storage device) that can retain programs and data even when a power is turned off. The ROM 505 stores programs and data of a BIOS, an OS setting, a network setting, or the like to be executed when starting up the computer 500. The RAM 504 is an example of a volatile memory (storage device) that temporarily stores programs and data.

The CPU 506 is an arithmetic device that reads programs and data from a storage device, such as the ROM 505, the HDD 508, or the like, into the RAM 504, and executes processes to perform the control and function of the entire computer 500. The information processing device 12 according to the present embodiment can implement various functions which will be described later. A description of the hardware configuration of the annealing type computer 10 will be omitted.

### <Configuration>

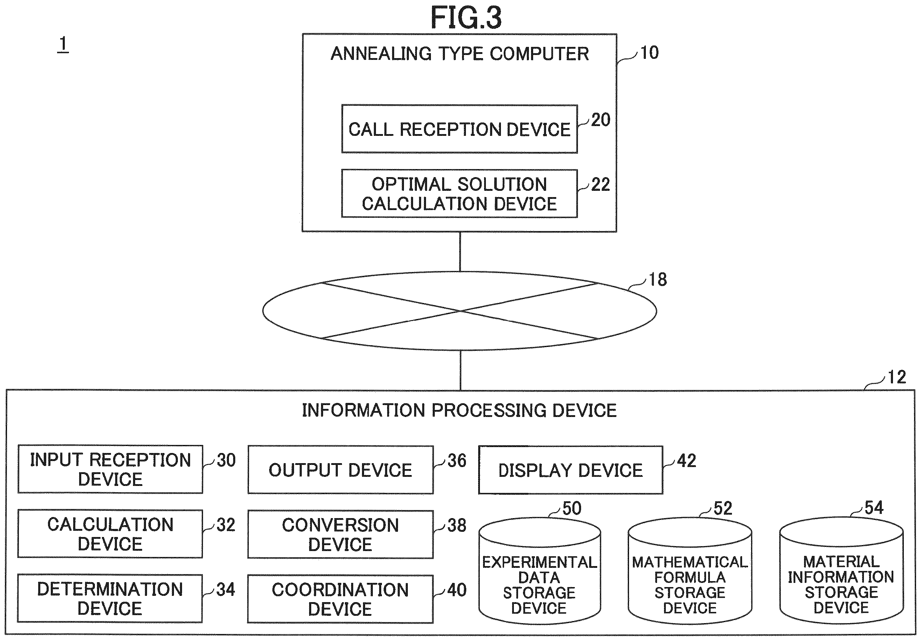

A configuration of the information processing system 1 according to the present embodiment will be described. FIG. 3 is a configuration diagram of an example of the information processing system according to the present embodiment. In the configuration diagram of FIG. 3, parts unnecessary for the description of the present embodiment are omitted, as appropriate.

The annealing type computer 10 of the information processing system 1 illustrated in FIG. 3 includes a call reception device 20, and an optimal solution calculation device 22. In addition, the information processing device 12 includes an input reception device 30, a calculation device 32, a determination device 34, an output device 36, a conversion device 38, a coordination device 40, a display device 42, an experimental data storage device 50, a mathematical formula storage device 52, and a material information storage device 54.

The input reception device 30 is an input interface configured to receive an input of information required for causing the annealing type computer 10 to solve the combinatorial optimization problem that is reduced to the Ising model, from the user. The information necessary for causing the annealing type computer 10 to solve the combinatorial optimization problem that is reduced to the Ising model, includes information on a function to be converted into the Ising model. The function to be converted into the Ising model is described so as to include one or more explanatory variables (an explanatory variable group).

The calculation device 32 calculates a relationship between one or more explanatory variables and the physical property of the mixed material, by a trained machine learning model described using one or more explanatory variables of the function to be converted into the Ising model. In addition, the explanatory variable used for describing the machine learning model is preferably the characteristic of the mixed material describable by a weighted average of a ratio of a material composition. It is assumed that the machine learning model is trained of a relationship between the characteristic of the mixed material describable by the weighted average of the ratio of the material composition and the physical property of the mixed material, using experimental data.

The determination device 34 determines an optimal value and a tolerable variation width of the one or more explanatory variables for the target physical property, based on the relationship between the one or more explanatory variables and the physical property of the mixed material calculated by the calculation device 32, as will be described later.

The output device 36 outputs the optimal value of the one or more explanatory variables determined by the determination device 34, as a target value of the one or more explanatory variables of the function to be converted into the Ising model. The output device 36 also outputs a weighting coefficient of the one or more explanatory variables of the function to be converted into the Ising model based on the tolerable variation width of the one or more explanatory variables determined by the determination device 34, as will be described later.

The conversion device 38 converts the function, to which the target value of the one or more explanatory variables and the weighting coefficient of the one or more explanatory variables output by the output device 36 are substituted, into the Ising model in a data format utilizable by the annealing type computer 10.

The coordination device 40 transmits the Ising model converted by the conversion device 38 to the annealing type computer 10. Further, the coordination device 40 receives the optimal solution calculated by the annealing type computer 10.

The display device 42 displays the optimal solution received by the coordination device 40 on the display device 502, to be checked by the user. The optimal solution displayed on the display device 502 is displayed as a mixing ratio of the mixed material, for example, which can be easily understood by the user.

The experimental data storage device 50 stores the experimental data utilized for training the machine learning model. The mathematical formula storage device 52 stores the function to be converted into the Ising model, and the machine learning model. The material information storage device 54 stores material information, such as the characteristics or the like of the material.

The call reception device 20 receives a call from the information processing device 12, and receives the Ising model converted into the utilizable data format from the information processing device 12. The optimal solution calculation device 22 searches for an optimal solution of the mixing ratio of the mixed material having a physical property asymptotically approaching the target value, by obtaining a state in which the energy (Hamiltonian) of the Ising model received by the call reception device 20 becomes a minimum. The call reception device 20 transmits the searched optimal solution to the information processing device 12.

The configuration diagram of FIG. 3 is merely an example. Various configurations of the information processing system 1 according to the present embodiment are conceivable.

### <Processes>

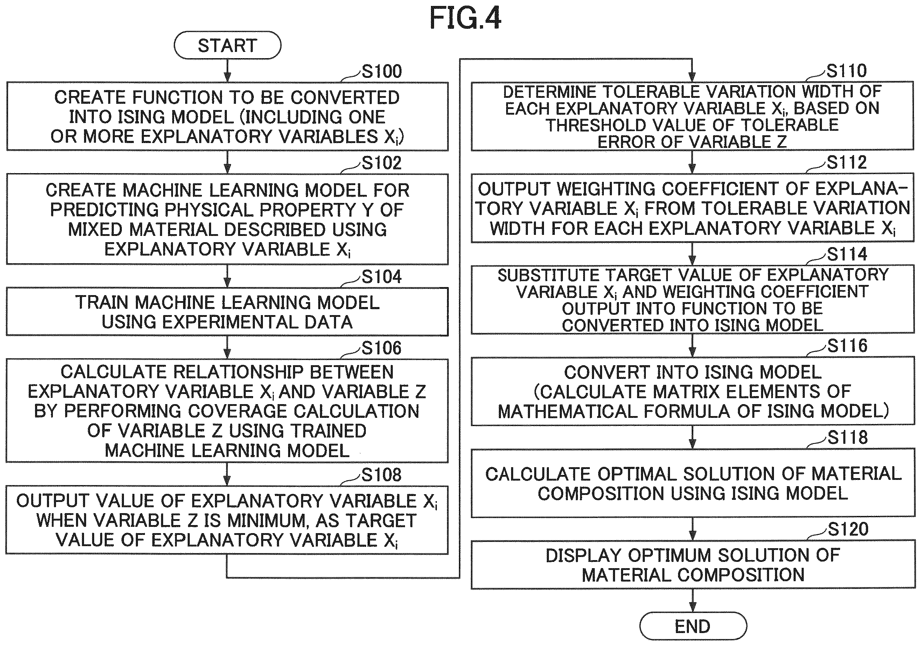

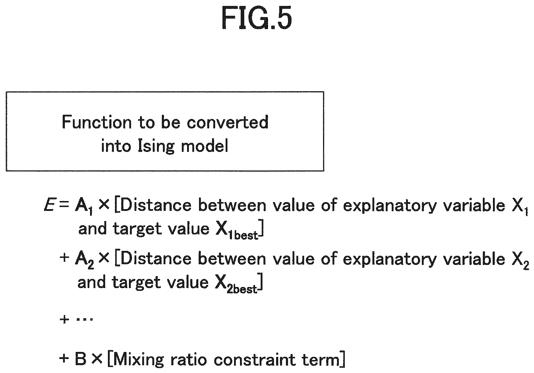

FIG. 4 is a flow chart illustrating an example of a processing procedure of a material composition searching method using the information processing system according to the present embodiment. In step S100, the user creates a function to be converted into an Ising model. The function to be converted into the Ising model has a configuration illustrated in FIG. 5, for example.

FIG. 5 is an explanatory diagram of an example of the function to be converted into the Ising model. The function to be converted into the Ising model, illustrated in FIG. 5, includes the value of one or more explanatory variables Xᵢ (i = 1, 2, ...) . X_{ibest} denotes a target value of the explanatory variable Xᵢ. The explanatory variable Xᵢ is the characteristic of the mixed material, such as a HSP value, a molecular weight, or the like described by the weighted average of the ratio of the material composition.

In FIG. 5, terms other than a last term have values that are smaller as the explanatory variable Xᵢ approaches the target value X_{ibest}. In addition, a mixing ratio constraint term is a term that becomes "0" when a sum of the mixing ratios of the materials is "1000". Further, in the function to be converted into the Ising model in FIG. 5, Aᵢ denotes a weighting coefficient of the explanatory variable Xᵢ. B denotes a weighting coefficient of the mixing ratio constraint term.

Because the annealing type computer 10 obtains the state in which the function of FIG. 5 becomes the minimum, as an optimization result, for example, the annealing type computer 10 calculates a state in which the explanatory variable Xᵢ is close to the target value X_{ibest} and the sum of the mixing ratios of the materials is "100%", as an optimal solution of the material composition.

In the present embodiment, the information processing device 12 is caused to output the target value X_{ibest} and the weighting coefficient Aᵢ, which were conventionally adjusted by the user, so as to support the creation of the function to be converted into the Ising model.

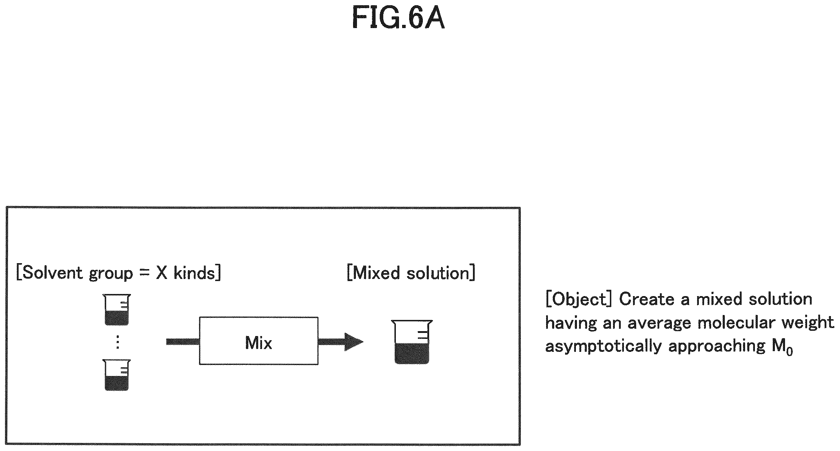

FIG. 6A through FIG. 6C are explanatory diagrams of an example of the problems to be solved as a combinatorial optimizing problem. FIG. 6A illustrates that the object is to mix a plurality of solvents (solvent group) having known molecular weights to create a mixed solution having an average molecular weight asymptotically approaching (approximating) M₀.

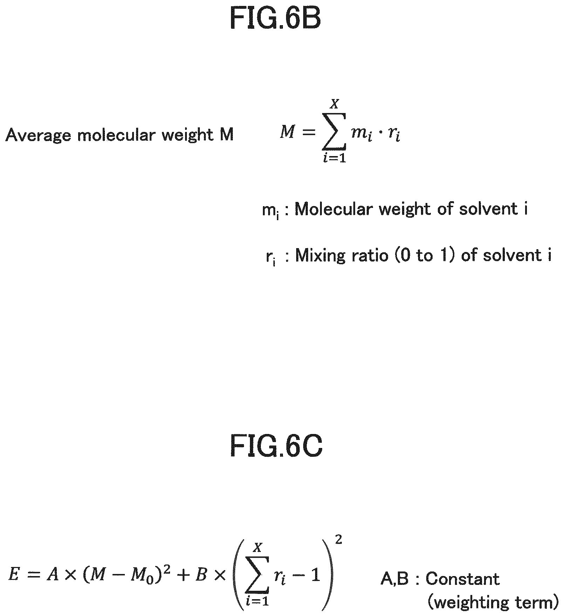

An average molecular weight M of the mixed solvent, which is a mixture of the solvent group, can be calculated by a formula illustrated in FIG. 6B. Further, a function that assumes a smaller value as the average molecular weight M of the mixed solvent, which is a mixture of the solvent group, asymptotically approaches M₀, can be described as illustrated in FIG. 6C, for example. The average molecular weight M and M₀ are examples of the characteristics of the mixed solvent describable by the weighted average of the mixing ratio of the mixed solvent, which is the mixture of the solvent group.

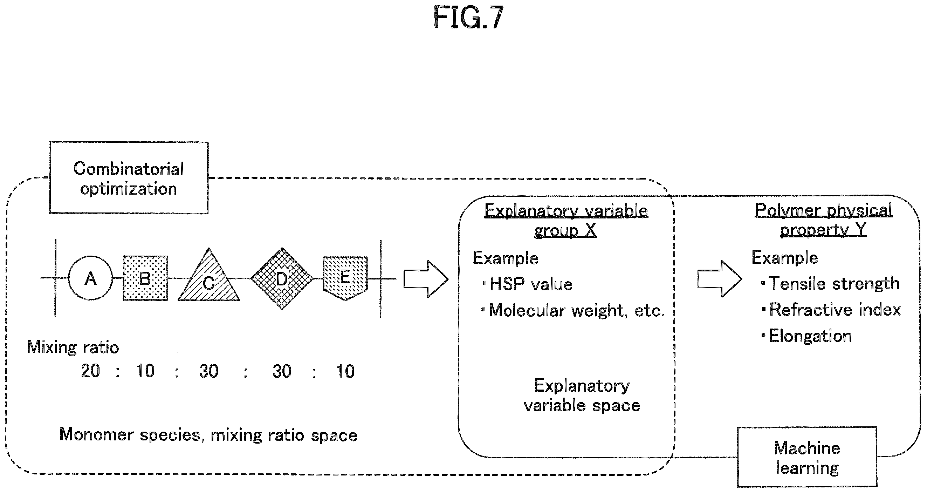

Returning to FIG. 4, in step S102, the user creates a machine learning model for predicting a physical property Y of the mixed material that are described using the explanatory variable Xᵢ included in the function to be converted into the Ising model. A process of step S102 will be described with reference to FIG. 7.

FIG. 7 is a diagram for explaining coordination between combinatorial optimization and machine learning. FIG. 7 illustrates an example of solving an optimal mixing ratio of monomer species for creating a polymer having the physical property Y, as a combinatorial optimization problem.

The function to be converted into the Ising model for solving the combinatorial optimization problem of FIG. 7 includes an explanatory variable group X describable by the weighted average of the mixing ratio of the monomer species, as described with reference to FIG. 5 or the like. Accordingly, a relationship between the explanatory variable group X of the polymer and the physical property Y of the polymer can be obtained by creating a machine learning model for predicting the physical property of the polymer described using the explanatory variable group X of the polymer, and training the machine learning model using the experimental data.

As described above, in the present embodiment, a region of the explanatory variable group in an explanatory variable space for obtaining a polymer having a target physical property can be obtained as will be described later, by the machine learning model trained of the relationship between the explanatory variable group Xᵢ of the polymer and the physical property Y of the polymer. By obtaining the region of the explanatory variable group for obtaining the polymer having target physical property Y in the explanatory variable space of FIG. 7, the present embodiment determines the optimal value and the tolerable variation width of the explanatory variable Xᵢ illustrated in FIG. 5 as will be described later, to be utilized for outputting the target value X_{ibest} of the explanatory variable Xᵢ and the weighting coefficient Aᵢ.

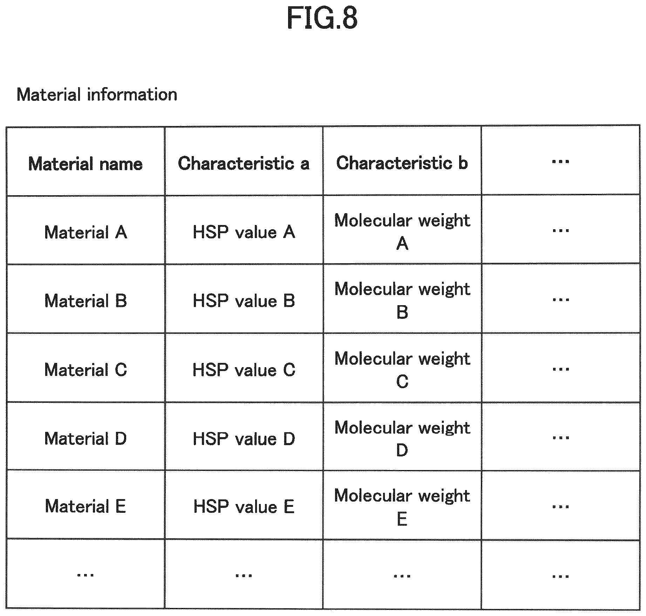

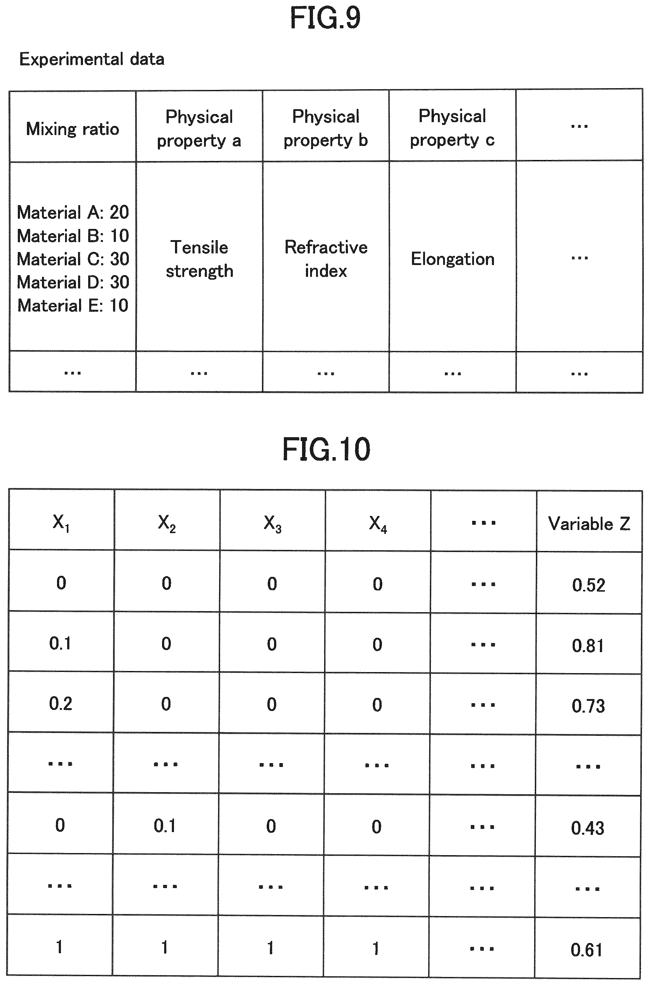

Returning to FIG. 4, in step S104, the user causes the machine learning model created in step S102 to train using experimental data. The type of the machine learning model is not particularly limited, and LASSO, random forest, or the like can be utilized. For example, material information illustrated in FIG. 8 and experimental data illustrated in FIG. 9 are utilized for the training of the machine learning model.

FIG. 8 is a configuration diagram of an example of the material information. In the material information of FIG. 8, values of the characteristics are set for each material. FIG. 9 is a configuration diagram of an example of the experimental data. In the experimental data of FIG. 9, the mixing ratio of the mixed material, and the values of the physical properties of the mixed material mixed with the mixing ratio, are set. The explanatory variable ᵢ can be calculated based on the material information of FIG. 8 and the mixing ratio of the mixed material illustrated in FIG. 9.

Returning to FIG. 4, in step S106, the user creates a variable Z for which a smaller value is output for the mixed material that asymptotically approaches the target physical property, using the machine learning model trained in step S104. The calculation device 32 of the information processing device 12 determines grid points for performing a coverage calculation in the explanatory variable space, for example, and performs a coverage calculation of the variable Z to calculate the relationship between the explanatory variable Xᵢ and the variable Z such as that illustrated in FIG. 10.

FIG. 10 is a diagram of an example illustrating the relationship between the calculated explanatory variable Xᵢ and the calculated variable Z. The smaller the value of the variable Z, the closer the mixed material approaches the target physical property. Accordingly, the explanatory variable Xᵢ corresponding to the variable Z having the minimum value illustrated in FIG. 10 becomes the optimal value of the explanatory variable Xᵢ. In addition, by setting a threshold value of a tolerable error from the minimum value of the variable Z (for example, tolerating an error of c% from the value of the variable Z), a plurality of values of the variable Z within the threshold value of the tolerable error from the minimum value of the variable Z can be selected.

Returning to FIG. 4, in step S108, the determination device 34 determines the value of the explanatory variable Xᵢ when the variable Z has the minimum value, as the optimal value of the explanatory variable Xᵢ. The output device 36 outputs the determined optimal value of the explanatory variable Xᵢ, as the target value X_{ibest} of the explanatory variable Xᵢ.

Moreover, in step S110, the determination device 34 selects a plurality of values of the variable Z within the threshold value of the tolerable error from the minimum value of the variable Z, and determines the tolerable variation width of the explanatory variable Xᵢ using the value of the explanatory variable Xᵢ of the selected variable Z.

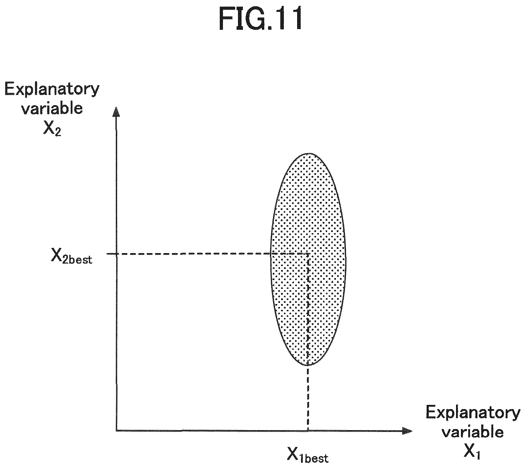

FIG. 11 is a diagram of an example for explaining the optimal value and the tolerable variation width of the explanatory variable Xᵢ. FIG. 11 illustrates an example in which the explanatory variable Xᵢ is two dimensional. An optimal value of an explanatory variable X₁ has a value X_{1best}. In addition, an optimal value of an explanatory variable X₂ has a value X_{2best}.

An elliptical region illustrated in FIG. 11 can be plotted using the values of the explanatory variable X₁ and the explanatory variable X₂ of the plurality of variables Z within the threshold value of the tolerable error from the minimum value of the variable Z. The elliptical region illustrated in FIG. 11 is the explanatory variable region of the mixed material asymptotically approaching the target physical property. Although FIG. 11 illustrates an example in which the explanatory variable region of the mixed material asymptotically approaching the target physical property is elliptical, the explanatory variable region is not limited to the elliptical region.

The determination device 34 determines the tolerable variation width for each of the explanatory variable X₁ and the explanatory variable X₂, from the elliptical region illustrated in FIG. 11. For example, the tolerable variation width of the explanatory variable X₁ is a length of a minor axis of the elliptical region illustrated in FIG. 11. For example, the tolerable variation width of the explanatory variable X₂ is a length of a major axis of the elliptical region illustrated in FIG. 11.

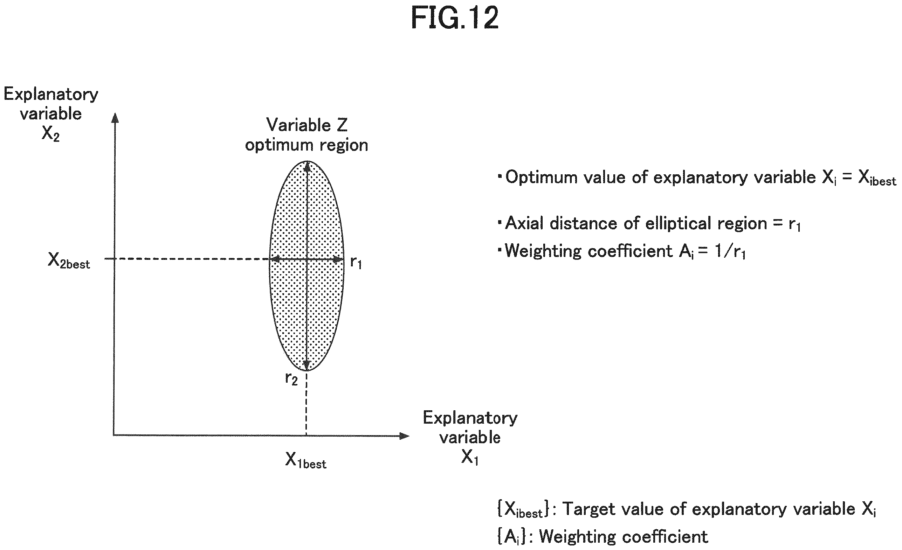

Returning to FIG. 4, in step S112, the output device 36 determines the weighting coefficient Aᵢ of the explanatory variable Xᵢ from the determined tolerable variation width for each explanatory variable Xᵢ by a method as illustrated in FIG. 12, for example, and outputs the determined weighting coefficient Aᵢ.

FIG. 12 is an explanatory diagram of an example of a process for determining the weighting coefficient Aᵢ of the explanatory variable Xᵢ. For example, the weighting coefficient Aᵢ of the explanatory variable Xᵢ can be calculated from 1/rᵢ, where rᵢ denotes an axial distance of the elliptical region. In the example of FIG. 12, the weighting coefficient A₁ of the explanatory variable X₁ is 1/r₁. In the example of FIG. 12, the weighting coefficient A₂ of the explanatory variable X₂ is 1/r₂.

For example, in the explanatory variable region (elliptical region) of the mixed material asymptotically approaching the target physical property illustrated in FIG. 12, a sensitivity of the explanatory variable X₁ with respect to the target physical property is higher than a sensitivity of the explanatory variable X₂, and thus, an importance of the explanatory variable Xᵢ is determined as the explanatory variable X₁ > the explanatory variable X₂, and the weighting coefficients are output such that the weighting coefficient A₁ > the weighting coefficient A₂. In the example of FIG. 12, the weighting coefficient Aᵢ is output such that the explanatory variable Xᵢ having a larger tolerable variation width (larger variance) has a smaller weighting coefficient Aᵢ, and the explanatory variable Xᵢ having a smaller tolerable variation width (smaller variance) has a larger weighting coefficient Aᵢ.

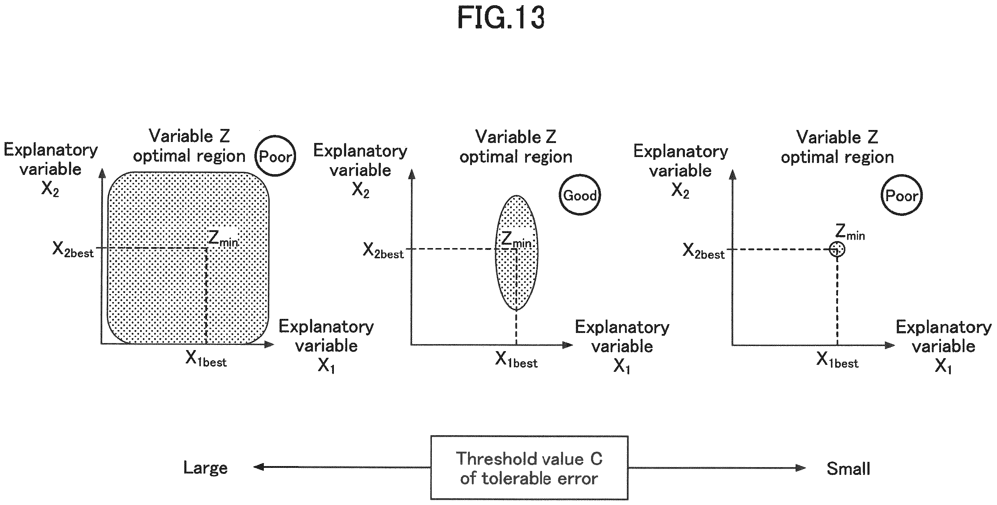

The explanatory variable region of the mixed material asymptotically approaching the target physical property illustrated in FIG. 12 changes shape depending on the threshold value of the tolerable error from the minimum value of the variable Z. FIG. 13 is a diagram of an example for explaining a relationship between a threshold value C of the tolerable error from the minimum variable Z and the shape of the explanatory variable region of the mixed material asymptotically approaching the target physical property.

If the threshold value C of the tolerable error from the minimum variable Z is too large, the shape of the explanatory variable region of the mixed material asymptotically approaching the target physical property becomes too large, as illustrated in a graph on the left side in FIG. 13, for example, and it may not be possible to effectively determine the sensitivity of the explanatory variable X₁ and the sensitivity of the explanatory variable X₂ with respect to the target physical property. On the other hand, if the threshold value C of the tolerable error from the minimum variable Z is too small, the shape of the explanatory variable region of the mixed material asymptotically approaching the target physical property becomes too small, as illustrated in a graph on the right side in FIG. 13, and it may not be possible to effectively determine the sensitivity of the explanatory variable X₁ and the sensitivity of the explanatory variable X₂ with respect to the target physical property.

Accordingly, it is desirable to set the threshold value C of the tolerable error from the minimum variable Z so that the difference of the explanatory variables Xᵢ can be identified, as illustrated in a graph in the center of FIG. 13. The threshold value C of the tolerable error from the minimum variable Z may be adjusted by the user with reference to the shape of the explanatory variable region of the mixed material asymptotically approaching the target physical property, or may be adjusted when the optimization result obtained by the annealing type computer 10 is determined as being insufficient.

Returning to FIG. 4, in step S114, the conversion device 38 substitutes the target value X_{ibest} of the explanatory variable Xᵢ output in step S108 and the weighting coefficient Aᵢ of the explanatory variable Xᵢ output in step S112 into the function to be converted into the Ising model. In step S116, the conversion device 38 converts the function, to which the target value X_{ibest} of the explanatory variable Xᵢ and the weighting coefficient Aᵢ of the explanatory variable Xᵢ are substituted, into the Ising model (calculates matrix elements of the mathematical formula of the Ising model).

Techniques for converting a function into a quadratic unconstrained binary optimization (QUBO) format of an evaluation function, and converting a function into an Ising model in a data format utilizable by the annealing type computer 10, are provided as a Web API or the like, and are existing techniques.

For example, the conversion device 38 expands the function to which the target value X_{ibest} of the explanatory variable Xᵢ and the weighting coefficient Aᵢ of the explanatory variable Xᵢ are substituted, and calculates matrix elements Q_{i,j} of the Ising model, and the coordination device 40 transmits the matrix elements Q_{i,j} calculated by the conversion device 38 to the annealing type computer 10, as parameters of the Ising model.

In step S118, the optimal solution calculation device 22 of the annealing type computer 10, which receives the parameters of the Ising model, searches for the optimal solution of the mixing ratio of the mixed material with which the function of FIG. 5 becomes a minimum, for example. The call reception device 20 transmits information indicating the searched optimal solution to the information processing device 12.

In step S120, the display device 42 converts the information (bit information of the annealing type computer 10) received by the coordination device 40 as the optimal solution into information, such as the mixing ratio of the mixed material or the like, which can be easily understood by the user, and outputs the converted information. For example, the display device 42 displays material names of the materials included in the mixed solvent of the optimal solution, and the mixing ratio of the materials.

In the present embodiment, it is possible to omit the input of the target value X_{ibest} and the weighting coefficient Aᵢ of the explanatory variable, which was conventionally required of the user in order to cause the annealing machine to solve the combinatorial optimization problem reduced to the Ising model.

The mixing ratio of the mixed material searched as the optimal solution can be used to generate the mixed material by designating the materials to be mixed and the mixing ratio. For example, the optimal solution can be used for controlling a mixed material generation device. In addition, the physical property of the mixed material generated by the mixed material generation device can be evaluated by an evaluation device.

Accordingly, the information of the mixed material searched as the optimal solution can be compared with the physical property of the mixed material generated by the mixed material generation device by designating the information of the mixed material, and the accuracy of the search for the optimal solution can be improved by feeding back the result of the comparison.

As described above, according to the information processing system 1 of the present embodiment, it is possible to reduce the time and effort for creating the Ising model for causing the annealing type computer 10 to solve the combinatorial optimization problem of the material composition asymptotically approaching the target physical property.

Although the present embodiment is described above, it will be understood that various changes in form and detail may be made without departing from the spirit and scope of the appended claims.

Although the present embodiment is described above, it will be understood that various variations in form and detail may be made without departing from the spirit and scope of the appended claims. Although the present invention is described above based on the embodiments, the present invention is not limited to the above described embodiments, and various modifications can be made within the scope defined in the claims. This application is based upon and claims priority to Japanese Patent Application No. 2022-031084 filed on March 1, 2022, the entire contents of which are incorporated herein by reference.

### DESCRIPTION OF REFERENCE NUMERALS

- 1:: Information processing system
- 10:: Annealing type computer
- 12:: Information processing device
- 18:: Communication network
- 20:: Call reception device
- 22:: Optimal solution calculation device
- 30:: Input reception device
- 32:: Calculation device
- 34:: Determination device
- 36:: Output device
- 38:: Conversion device
- 40:: Coordination device
- 42:: Display device
- 50:: Experimental data storage device
- 52:: Mathematical formula storage device
- 54:: Material information storage device

## Claims

1. An information processing device configured to support creation of an Ising model for causing an annealing type computer to solve a combinatorial optimization problem of a material composition asymptotically approaching a target physical property, **characterized in that** there are provided:
a calculation device configured to calculate a relationship between one or more explanatory variables of a function to be converted into the Ising model and a physical property of a mixed material, by a trained machine learning model that is for predicting the physical property of the mixed material and is described using the one or more explanatory variables;
a determination device configured to determine an optimal value and a tolerable variation width of the one or more explanatory variables for the target physical property, based on the relationship between the one or more explanatory variables and the physical property of the mixed material; and
an output device configured to output the determined optimal value of the one or more explanatory variables as a target value of the one or more explanatory variables of the function, and output a weighting coefficient of the one or more explanatory variables of the function based on the determined tolerable variation width of the one or more explanatory variables.

2. The information processing device as claimed in claim 1, **characterized in that** the one or more explanatory variables are characteristics of the mixed material describable by a weighted average of a ratio of the material composition.

3. The information processing device as claimed in claim 1 or 2, **characterized in that** the output device outputs a smaller weighting coefficient for the explanatory variable having a larger tolerable variation width, and outputs a larger weighting coefficient for the explanatory variable having a smaller tolerable variation width.

4. The information processing device as claimed in any one of claims 1 to 3, **characterized in that** the determination device determines the tolerable variation width for each of the one or more explanatory variables, based on a threshold value of a tolerable error of the target physical property.

5. The information processing device as claimed in any one of claims 1 to 4, **characterized in that** the machine learning model is trained of a relationship between a characteristic of the mixed material describable by a weighted average of a ratio of the material composition and the physical property of the mixed material, using experimental data.

6. The information processing device as claimed in any one of claims 1 to 5, **characterized in that** there is further provided:
a conversion device configured to convert the function, to which the target value of the one or more explanatory variables and the weighting coefficient of the one or more explanatory variables output by the output device are substituted, into the Ising model.

7. An information processing system including an annealing type computer using an Ising model, and an information processing device configured to support creation of the Ising model for causing the computer to solve a combinatorial optimization problem of a material composition asymptotically approaching a target physical property, **characterized in that** there are provided:
a calculation device configured to calculate a relationship between one or more explanatory variables of a function to be converted into the Ising model and a physical property of a mixed material, by a trained machine learning model that is for predicting the physical property of the mixed material and is described using the one or more explanatory variables;
a determination device configured to determine an optimal value and a tolerable variation width of the one or more explanatory variables for the target physical property, based on the relationship between the one or more explanatory variables and the physical property of the mixed material;
an output device configured to output the determined optimal value of the one or more explanatory variables as a target value of the one or more explanatory variables of the function, and output a weighting coefficient of the one or more explanatory variables of the function based on the determined tolerable variation width of the one or more explanatory variables;
a conversion device configured to convert the function, to which the target value of the one or more explanatory variables and the weighting coefficient of the one or more explanatory variables output by the output device are substituted, into the Ising model;
an optimal solution calculation device configured to calculate an optimal solution of the material composition asymptotically approaching the target value, using the Ising model; and
a display device configured to display the optimal solution of the material composition asymptotically approaching the target value.

8. A program which causes an information processing device configured to support creation of an Ising model for causing an annealing type computer to solve a combinatorial optimization problem of a material composition asymptotically approaching a target physical property, to function as:
a calculation device configured to calculate a relationship between one or more explanatory variables of a function to be converted into the Ising model and a physical property of a mixed material, by a trained machine learning model that is for predicting the physical property of the mixed material and is described using the one or more explanatory variables;
a determination device configured to determine an optimal value and a tolerable variation width of the one or more explanatory variables for the target physical property, based on the relationship between the one or more explanatory variables and the physical property of the mixed material; and
an output device configured to output the determined optimal value of the one or more explanatory variables as a target value of the one or more explanatory variables of the function, and output a weighting coefficient of the one or more explanatory variables of the function based on the determined tolerable variation width of the one or more explanatory variables.

9. A material composition searching method for an information processing system including an annealing type computer using an Ising model, and an information processing device configured to support creation of the Ising model for causing the computer to solve a combinatorial optimization problem of a material composition asymptotically approaching a target physical property, **characterized in that** there are provided:
calculating a relationship between one or more explanatory variables of a function to be converted into the Ising model and a physical property of a mixed material, by a trained machine learning model that is for predicting the physical property of the mixed material and is described using the one or more explanatory variables;
determining an optimal value and a tolerable variation width of the one or more explanatory variables for the target physical property, based on the relationship between the one or more explanatory variables and the physical property of the mixed material;
outputting the determined optimal value of the one or more explanatory variables as a target value of the one or more explanatory variables of the function, and output a weighting coefficient of the one or more explanatory variables of the function based on the determined tolerable variation width of the one or more explanatory variables;
converting the function, to which the target value of the one or more explanatory variables and the weighting coefficient of the one or more explanatory variables output by the output device are substituted, into the Ising model;
calculating an optimal solution of the material composition asymptotically approaching the target value, using the Ising model; and
displaying the optimal solution of the material composition asymptotically approaching the target value.
